# EUROPEAN PATENT APPLICATION

(11) **EP 2 357 239 A1**
(43) Date of publication of application: **17.08.2011**
(21) Application number: 09174538.0
(22) Date of filing: 29.10.2009
(51) Int. Cl.: C12N 15/82, C12N 15/55, C12N 5/14

(54) **Methods and means for a selectable marker system in plants**

(71) Applicant: Universität zu Köln, 50923 Köln (DE)
(72) Inventor: Kunz, Hans-Henning, 50677 Köln (DE); Flügge, Ulf-Ingo, 50977 Köln (DE); Gierth, Markus, 50321 Brühl (DE); Neuhaus, Ekkehard, 67659 Kaiserslautern (DE); Möhlmann, Thorsten, 67735 Mehlbach (DE)
(74) Representative: Michalski Hüttermann & Partner Patentanwälte

(57) **Abstract**

Transformed plants that are capable of germinating in the presence of an externally applied chemical, nucleic acid sequences, vectors, and plasmids therefore, plant cells and plants transformed with such nucleic acid sequences, vectors and plasmids, a transient selectable marker system for use in the germination stages of plant development.

## Description

The present invention relates to the provision of plants and plant cells and a transient selectable marker system for use therein. In particular, the invention relates to nucleic acid sequences, vectors, and plasmids therefore, plant cells and plants transformed with such nucleic acid sequences, vectors and plasmids, a transient selectable marker system for use in the germination stages of plant development, methods and uses.

Numerous selection marker systems are known in the art. Such systems are used to identify transgenic plants that carry certain traits of interest and are generally based on the use of extrinsic markers derived from microorganisms in combination with constitutive promoters or other high expression promoters that do not display a natural transient expression profile in the plant cell. Such promoters have been used for high levels of expression of introduced polynucleotide sequences of choice. The use of selection systems based on constitutive expression systems has uncomfortable implications for biosafety. There exists a need for more reliably safe selection marker systems for use in plants.

It appears that of the prior art selection marker systems none seem to have been based on plant-intrinsic gene or plant-intrinsic polynucleotide sequences that drive expression from plant intrinsic promoters of introduced polynucleotide sequences that are capable of providing a tolerance to selection agents. An example of a system that uses a constitutive promoter (CaMV35S) is the trehalose-6-phosphate synthase system that employs a plant synthase taken from *Arabidopsis thaliana* and which is used to transform tobacco. Transformants are identified as green seedlings that are grown on a high glucose medium (Leyman B., et al Journal of Biotechnology 121 (2006) pp. 309-317). This system relies on the use of a selection agent (glucose) which growing transgenic seedlings display a tolerance to but which tolerance is absent in wild type plants. Such a system suffers from the disadvantage that the constructs used by Leyman B. et al *supra* are driven by a constitutive promoter (the CaMV35S promoter) that causes high expression in all tissues and developmental stages and may thus lead to undesirable impacts on metabolism and pleiotropic effects in general. Furthermore, the Applicant considers that the selection marker system described by Leyman et al *supra,* is likely to be inefficient since trehalose-6-phosphate synthase acts as an important signal molecule for developmental processes and is involved in the regulation of starch and carbon metabolism.

The present inventors have found that by designing a system in which the expression of a polynucleotide sequence of interest when expressed as a polypeptide or protein sequence in a plant cell confers a tolerance to a plant cell to an applied chemical or a tolerance to a plant to an applied chemical, such as a nucleosidase polynucleotide sequence, for example, a uridine ribohydrolase 1 (URH1) DNA sequence that is driven by a substantially germination phase specific, transiently active plant promoter, such as one that is active in the glyoxylate cycle, a reliable germination phase specific selectable marker system for use in plants and plant cells has been achieved. For the purposes of the invention, a "substantially germination phase specific transiently active promoter" is one that displays higher, detectable promoter activity in plant cells during the germination phase of a plant relative to the level of promoter activity of the same kind that may be found in plant cells that are not in the germination phase. Thus, the "substantially germination phase specific transiently active promoter" of the invention displays little, if any, detectable promoter activity in plant cells that are outside of the germination phase of the plant. Suitable promoters of the aforementioned kind include the malate synthase promoter or a functional part thereof, or the isocitrate lyase promoter or a functional part thereof. By designing and employing such a system as outlined above, an efficient selection system has been achieved. Such a selection marker system is useful for selecting transformants at a particular phase, that is to say, the germination phase, of a plant cycle and provides plant cells and plants comprising the aforesaid sequences with a tolerance towards an applied chemical, such as substituted pyridine ring structures comprising a halogen substituent, which tolerance is substantially absent in wild type plants and plant cells. Furthermore, such combinations of sequences, that is to say, an intrinsic plant promoter that is substantially operable in the germination phase of a plant in combination with a selectable marker polynucleotide sequence of choice as outlined herein are substantially inactive outside of the germination phase of development of a plant, as alluded to herein. The importance of this finding is that the expression of selection systems of the invention can be tightly controlled within a specific time frame namely, the germination phase of a plant. Thus, the selection system of the present invention is likely to be safer and more controllable than other selection systems employed in the art.

For the purposes of the present invention the terms "plant cell" and "plant cells" are used interchangeably, unless context demands otherwise. Furthermore, for the purposes of the present invention, "chemical tolerance" means that plant cells or plants that express functional polypeptides from polynucleotide sequences employed in the invention remain viable in the presence of chemicals that are employed at concentrations of use in selection systems of the invention, whereas plant cells or plants that do not comprise such polynucleotide sequences become substantially intolerant in the presence of such chemicals, and lose viability.

There exists a need for alternative, safer selection systems for use in methods for the transformation of plant cells over those of the prior art.

The basis for the present invention, which does not appear to have been realised in the prior art, is to introduce into a plant genome at least a nucleotide sequence of interest that is capable of providing a tolerance to an externally applied chemical stimulus, such as a nucleosidase polynucleotide sequence of interest, for example, a plant uridine ribohydrolase sequence under the control of an endogenous plant promoter. Typically, such an endogenous plant promoter is substantially, if not completely active in plant cells during the germination phase of a plant. Such sequences are introduced into the nucleus of a plant host cell where it is expressed as RNA. The nucleotide sequence of a polynucleotide of interest in the invention may be further modified to eliminate cryptic splicing sites, thus improving expression in the plant cell.

The present invention also relates to the production of transgenic plant cells and transgenic plants comprising an introduced polynucleotide sequence under operational control of a non-constitutive promoter that is capable of driving expression of the polynucleotide sequence while the plant is in the germination phase. Furthermore, any selected polynucleotide sequence for use in the invention, for example, a nucleosidase sequence, is one that is capable of conferring a tolerance to a plant cell to an exogenously applied chemical. Similarly, a plant comprising chemical tolerant cells that displays a tolerance to an exogenously applied chemical as alluded to above that is growing and in the germination phase, is also encompassed within the ambit of the invention. Such exogenously applied chemicals include any applied chemical commonly employed in the art in selection systems, such as those selected from spectinomycin, streptomycin, kanamycin, neomycin, hygromycin, puramycin, phosphinotricin, chlorsulfuron, methotrexate, gentamycin, imidazolinones and glyphosate, or for example, from substituted pyridine ring structures that possess an halogen substituent selected from the group chlorine, fluorine, bromine and iodine, such as halouridine, for example, fluorouridine, or halouracil, for example, fluorouracil or haloorotic acid, for example, fluoroorotic acid.

According to the present invention there is provided a method of producing a transient tolerance in a plant cell to an externally applied chemical stimulus that comprises:
1) introducing into the said plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said plant cell is capable of providing a tolerance to the said externally applied chemical stimulus to the said plant cell;
2) growing said plant cell of (1) under conditions wherein said non-constitutive plant nuclear promoter drives expression of said isolated nucleic acid sequence;
3) selecting a plant cell of (2) wherein said isolated nucleic acid sequence is integrated into the nuclear genome;
4) growing the plant cell of (3) under conditions wherein said non-constitutive plant promoter drives the expression of said heterologous or exogenous polynucleotide sequence from said nucleic acid sequence.

Furthermore, there is provided a method of producing at least a heterologous or exogenous polynucleotide sequence that is capable of providing a tolerance to a plant to an externally applied chemical stimulus that comprises:
1) introducing into a regenerable plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said regenerable plant cell is capable of providing a tolerance to the said externally applied chemical stimulus to the said regenerable plant cell;
2) growing said plant cell of (1) under conditions wherein said non-constitutive plant nuclear promoter drives expression of said isolated nucleic acid sequence;
3) selecting a plant cell of (2) wherein said isolated nucleic acid sequence is integrated into the nuclear genome;
4) regenerating a plant from the plant cell of (3); and
5) growing the plant of (4) under conditions wherein said non-constitutive promoter expresses said heterologous or exogenous polypeptide from said isolated nucleic acid sequence.

The heterologous or exogenous polynucleotide sequence is selected typically from the group of polynucleotide sequences that code for functionally active proteins that confer tolerance to plant cells to such chemical agents as spectinomycin, streptomycin, kanamycin, neomycin, hygromycin, puramycin, phosphinotricin, chlorsulfuron, methotrexate, gentamycin, imidazolinones, glyphosate, and pyridine ring structures substituted with halogen such as fluorouridine, fluoroorotic acid and fluorouracil. Preferably, the heterologous or exogenous polynucleotide sequence is a nucleosidase sequence that confers tolerance to plant cells in the presence of a substituted pyridine ring structure substituted with halogen such as fluorouridine, fluoroorotic acid and fluorouracil. An example of a polynucleotide sequence suitable for use on the invention is a uridine ribohydrolase 1 sequence or a functional part thereof.

Preferably, the non-constitutive promoter of use in the invention is selected from tissue specific promoters that are operative in plant cells of a plant in its germination phase. Suitable promoter regions of use in the invention are plant nuclear promoter regions that are selected from the plant malate synthase promoter region or a functional part thereof and a plant isocitrate lyase promoter region or a functional part thereof. Preferably, the plant nuclear promoter region of use in the invention is the malate synthase promoter region of a plant or a functional part thereof. Specific promoter regions may be selected from the *Arabidopsis thaliana* malate synthase promoter (At5g03860) (SEQ ID No.3) or a functional part thereof and the Arabidopsis thaliana isocitrate lyase promoter (At3g21720)(SEQ ID No.2) or a functional part thereof.

Preferably, the isolated nucleic acid sequence of use in the invention codes for a functionally active protein and is a plant uridine ribohydrolase sequence that encodes an RNA from which a functionally active uridine ribohydrolase protein may be translated. The said nucleic acid sequence may optionally include a second isolated nucleic acid sequence under control of a promoter of use in the present invention that when expressed provides a protein selected from an herbicide tolerance protein or a functionally active part thereof or an antibiotic tolerance protein, or a functionally active part thereof. Examples of plant uridine ribohydrolase nucleic acid sequences of use in the invention are ones corresponding to functionally active proteins selected from the *Arabidopsis thaliana* sequence At2g36310, the *Zea mays* sequence ACF80359, the *Oryza sativa* sequence Os08g05579;XP483754, and the *Vitis vinifera* sequence, CAO15947. Examples of the heterologous or exogenous polypeptides of use in plant cells and germinating plants of the invention may be selected from the group consisting of the *Arabidopsis thaliana* sequence At2g36310 or a functionally active part thereof, the *Zea mays* sequence ACF80359 or a functionally active part thereof, the *Oryza sativa* sequence Os08g05579;XP483754 or a functionally active part thereof, and the *Vitis vinifera* sequence, CAO15947, or a functionally active part thereof.

Examples of the externally applied chemical stimulus that may be presented to cells and germinating plants of the invention include substituted pyridine ring structures comprising a halogen, such as substituted pyridine ring compounds selected from the group fluorouridine, fluorouracil, and fluoroorotic acid.

Isolated polynucleotides of use in the invention include isolated polynucleotides that encode an heterologous or exogenous polynucleotide sequence operably linked to a non-constitutive plant nuclear promoter of use in the invention that drives expression in a plant cell and may be selected from any polynucleotide sequence of use in the invention and may be selected from the group of polynucleotide sequences that code for functionally active proteins that confer tolerance to plant cells to spectinomycin, streptomycin, kanamycin, neomycin, hygromycin, puramycin, phosphinotricin, chlorsulfuron, methotrexate, gentamycin, imidazolinones, glyphosate, and pyridine ring structures substituted with halogen such as fluorouridine, fluoroorotic acid and fluorouracil. Preferably, the isolated polynucleotide for use in the invention encodes an heterologous or exogenous polynucleotide sequence that is a nucleosidase nucleic acid sequence that comprises a first isolated nucleosidase nucleic acid sequence operably linked to a non-constitutive plant nuclear promoter that drives expression in a plant cell wherein the said nucleosidase nucleic acid sequence is selected from nucleosidase nucleic acid sequences that are substantially operative during the germination stages of a plant.

Preferably, the isolated polynucleotide is a nucleosidase nucleic acid sequence that is operably linked to a malate synthase promoter or a functional part thereof, or is operably linked to an isocitrate lyase promoter or a functional part thereof. Preferably, the nucleosidase nucleic acid sequence is a uridine ribohydrolase nucleic acid sequence from a plant. Preferably, the nucleosidase nucleic acid sequence is one that encodes a functionally active protein selected from the *Arabidopsis thaliana* sequence At2g36310 or functionally active parts thereof, the *Zea mays* sequence ACF80359 or functionally active parts thereof, the *Oryza sativa* sequence Os08g05579;XP483754 of functionally active parts thereof, and the *Vitis vinifera* sequence CAO15947 or functionally active parts thereof. Naturally, the person skilled in the art will understand that the isolated polynucleotide sequence of use in the invention may comprise genomic DNA or may comprise a cDNA component.

Included within the ambit of the invention there is included nucleic acid vectors suitable for transformation of a plant cell or a bacterial cell and including a polynucleotide as herein described. The nucleic acid vector of the invention may be suitable for transformation of a prokaryotic cell such as an *Agrobacterium* cell. Such nucleic acid vectors of the invention should also be suitable for transforming a plant cell of an oil seed bearing plant, such as one selected from the group oilseed bearing *Brassica* species, such as *Brassica napus, Zea mays* (maize=corn), *Glycine max* (soyabean), and *Helianthus* species (sunflower).

The cDNA's encoding a polynucleotide of the invention, such as a uridine ribohydrolase nucleotide sequence, contain at least one type of promoter that is operable in a plant cell during germination, for example, an isocitrate lyase promoter or a functional part thereof or a malate synthase promoter or a functional part thereof, operatively linked to a nucleic acid sequence or nucleic acid sequence component as herein defined and as provided by the present invention. As discussed, this enables control of expression of the polynucleotide of the invention. The invention also provides plants transformed with polynucleotide sequences or constructs and methods including introduction of such polynucleotide nucleic acid sequences or constructs into a plant cell and/or induction of expression of said first or second nucleic acid sequence or construct within a plant cell, e.g. by application of a suitable stimulus, such as an effective amount of applied exogenous chemical that is metabolised by a protein or polypeptide that is expressed from the promoter and is functionally able to confer a tolerance to a plant growing in the germination phase. A suitable system is exemplified by the malate synthase promoter that is operably linked to a nucleosidase such as URH1, in the presence of exogenously applied pyridine ring structures as outlined herein.

Naturally, the man skilled in the art will appreciate that terminator DNA sequences will be present in constructs used in the invention. A terminator is contemplated as a DNA sequence at the end of a transcriptional unit which signals termination of transcription. These elements are 3'-non-translated sequences containing polyadenylation signals, which act to cause the addition of polyadenylate sequences to the 3' end of primary transcripts. For expression in plant cells the nopaline synthase transcriptional terminator (A. Depicker et al., 1982, J. of Mol. & Applied Gen. 1:561-573) sequence serves as a transcriptional termination signal. Those skilled in the art are well able to construct vectors and design protocols for recombinant nucleic acid sequences or gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 2nd edition, Sambrook et al, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992. The disclosures of Sambrook et al. and Ausubel et al. are incorporated herein by reference. Specific procedures and vectors previously used with wide success upon plants are described by Bevan (Nucl. Acids Res. 12, 8711-8721 (1984)); and Guerineau and Mullineaux (1993) Plant transformation and expression vectors. In: Plant Molecular Biology Labfax (Croy RRD ed.) Oxford, BIOS Scientific Publishers, pp 121-148).

Naturally, the skilled addressee will appreciate that each introduced polynucleotide sequence of use in the invention will be under regulatory control of an intrinsic plant promoter, such as a plant malate synthase promoter or a functional part thereof or a plant isocitrate lyase promoter or a functional part thereof, and terminator. When two or more target proteins are destined to be produced from a single carrier RNA it is preferable if they are able to be readily separated, for example by binding to different protein-specific antibodies (monoclonal or polyclonal) in the harvesting phase of the plant cell culture system.

Selectable genetic markers may facilitate the selection of transgenic plants and these may consist of chimaeric genes that confer selectable phenotypes such as resistance to antibiotics such as spectinomycin, streptomycin, kanamycin, neomycin, hygromycin, puramycin, phosphinotricin, chlorsulfuron, methotrexate, gentamycin, imidazolinones and glyphosate. Other genetic markers include nucleosidases that are of use in the invention, such as those nucleosidases capable of regulating the uridine degradation pathway, for example the uridine ribohydrolase nucleic acid sequence responsible for metabolizing substituted pyridine ring structures as defined herein.

When introducing selected nucleic acid sequences according to the present invention into a cell, certain considerations must be taken into account, well known to those skilled in the art. The nucleic acid to be inserted should be assembled within a construct, which contains effective regulatory elements, which will drive transcription. There must be available a method of transporting the construct into the cell. Once the construct is within the cell, integration into the endogenous chromosomal material either will or will not occur. Finally, as far as plants are concerned the target cell type must be such that cells can be regenerated into whole plants.

Plants transformed with DNA segments containing sequences of interest as provided herein may be produced by standard techniques, which are already known for the genetic manipulation of plants. DNA can be transformed into plant cells using any suitable technology, such as a disarmed Ti-plasmid vector carried by Agrobacterium exploiting its natural gene transfer ability (EP-A-270355, EP-A-0116718, NAR 12(22) 8711 -87215 1984), particle or micro projectile bombardment (US 5100792, EP-A-444882, EP-A-434616) microinjection (WO 92/09696, WO 94/00583, EP 331083, EP 175966, Green et al. (1987) Plant Tissue and Cell Culture, Academic Press), electroporation (EP 290395, WO 8706614) other forms of direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d) Physical methods for the transformation of plant cells are reviewed in Oard, 1991, Biotech. Adv. 9: 1-11. A particularly attractive method for cloning of genes into and back out of vectors, such as multiple vectors, is the Gateway® technology of Invitrogen Corporation (Gateway Technology Manual 2003, pp1-7), the teaching of which is incorporated herein.

Thus once a nucleic acid sequence or gene has been identified, it may be reintroduced into plant cells using techniques well known to those skilled in the art to produce transgenic plants of the appropriate phenotype.

*Agrobacterium* transformation is widely used by those skilled in the art to transform dicotyledonous species. Production of stable, fertile transgenic plants in almost all economically relevant monocot plants is also now routine:(Toriyama, et al. (1988) Bio/Technology 6, 1072-1074; Zhang, et al. (1988) Plant Cell Rep. 7, 379-384; Zhang, et al. (1988) Theor. Appl. Genet 76, 835-840; Shimamoto, et al. (1989) Nature 338, 274-276; Datta, et al. (1990) Bio/Technology 8, 736-740; Christou, et al. (1991) Bio/Technology 9, 957-962; Peng, et al. (1991) International Rice Research Institute, Manila, Philippines 563-574; Cao, et al. (1992) Plant Cell Rep. 11, 585-591; Li, et al. (1993) Plant Cell Rep. 12, 250-255; Rathore, et al. (1993) Plant Molecular Biology 21, 871-884; Fromm, et al. (1990) Bio/Technology 8, 833-839; Gordon-Kamm, et al. (1990) Plant Cell 2, 603-618; D'Halluin, et al. (1992) Plant Cell 4, 1495-1505; Walters, et al. (1992) Plant Molecular Biology 18, 189-200; Koziel, et al. (1993) Biotechnology 11, 194-200; Vasil, I. K. (1994) Plant Molecular Biology 25, 925-937; Weeks, et al. (1993) Plant Physiology 102, 1077-1084; Somers, et al. (1992) Bio/Technology 10, 1589-1594; WO92/14828). In particular, *Agrobacterium* mediated transformation is now a highly efficient alternative transformation method in monocots (Hiei et al. (1994) The Plant Journal 6, 271-282).

The generation of fertile transgenic plants has been achieved in the cereals rice, maize, wheat, oat, and barley (reviewed in Shimamoto, K. (1994) Current Opinion in Biotechnology 5, 158-162.; Vasil, et al. (1992) Bio/Technology 10, 667-674; Vain et al., 1995, Biotechnology Advances 13 (4): 653-671; Vasil, 1996, Nature Biotechnology 14 page 702). Wan and Lemaux (1994) Plant Physiol. 104: 37-48 describe techniques for generation of large numbers of independently transformed fertile barley plants.

Micro projectile bombardment, electroporation and direct DNA uptake are preferred where Agrobacterium is inefficient or ineffective. Alternatively, a combination of different techniques may be employed to enhance the efficiency of the transformation process, e.g. bombardment with Agrobacterium coated micro particles (EP-A-486234) or micro projectile bombardment to induce wounding followed by cocultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., Cell Culture and Somatic Cell Genetics of Plants, Vol. I, II and III, Laboratory Procedures and Their Applications, Academic Press, 1984, and Weiss Bach and Weiss Bach, Methods for Plant Molecular Biology, Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

The invention further encompasses a host cell transformed with vectors or constructs as set forth above, especially a plant or a microbial cell. Thus, a host cell, such as a plant cell, including nucleotide sequences of the invention as herein indicated is provided. Within the cell, the nucleotide sequence may be incorporated within the chromosome.

Thus host cells containing a heterologous polynucleotide or nucleic acid vectors as described herein also form an aspect of the present invention. Suitable host cells may include plant or bacterial cells. Suitable plant host cells include those selected from a plant from the group oilseed bearing *Brassica* species, such as *Brassica napus, Zea mays* (maize=corn), *Glycine max* (soyabean), and *Helianthus* species (sunflower). Such host cells may be comprised in a plant, a plant part or a plant propagule, or in a plant cell culture.

Also according to the invention there is provided a plant cell having incorporated into its genome at least a nucleotide sequence, particularly heterologous nucleotide sequences, as provided by the present invention under operative control of regulatory sequences for control of expression as herein described. The coding sequence may be operably linked to one or more regulatory sequences which may be heterologous or foreign to the nucleic acid sequences employed in the invention, such as those not naturally associated with the nucleic acid sequence(s) for its(their) expression. The nucleotide sequence according to the invention may be placed under the control of an externally inducible promoter to place expression under the control of the user. A further aspect of the present invention provides a method of making such a plant cell involving introduction of nucleic acid sequence(s) contemplated for use in the invention or a suitable vector including the sequence(s) contemplated for use in the invention into a plant cell and causing or allowing recombination between the vector and the plant cell genome to introduce the said sequences into the genome. The invention extends to plant cells containing a nucleotide sequence according to the invention as a result of introduction of the nucleotide sequence into an ancestor cell.

The term "heterologous" may be used to indicate that the gene/sequence of nucleotides in question have been introduced into said cells of the plant or an ancestor thereof, using genetic engineering, ie by human intervention. A transgenic plant cell, i.e. transgenic for the nucleotide sequence in question, may be provided. The transgene may be on an extra-genomic vector or incorporated, preferably stably, into the genome. A heterologous gene may replace an endogenous equivalent gene, i.e. one that normally performs the same or a similar function, or the inserted sequence may be additional to the endogenous gene or other sequence. An advantage of introduction of a heterologous gene is the ability to place expression of a sequence under the control of a promoter of choice, in order to be able to influence expression according to preference. Furthermore, mutants, variants and derivatives of the wild-type gene, e.g. with higher activity than wild type, may be used in place of the endogenous gene. Nucleotide sequences heterologous, or exogenous or foreign, to a plant cell may be non-naturally occurring in cells of that type, variety or species.

Thus, a nucleotide sequence may include a coding sequence of or derived from a particular type of plant cell or species or variety of plant, placed within the context of a plant cell of a different type or species or variety of plant. A further possibility is for a nucleotide sequence to be placed within a cell in which it or a homologue is found naturally, but wherein the nucleotide sequence is linked and/or adjacent to nucleic acid which does not occur naturally within the cell, or cells of that type or species or variety of plant, such as operably linked to one or more regulatory sequences, such as a promoter sequence, for control of expression.

A sequence within a plant or other host cell may be identifiably heterologous, exogenous or foreign.

Plants which include a plant cell according to the invention are also provided, along with any part or propagule thereof, seed, selfed or hybrid progeny and descendants. Particularly provided are transgenic crop plants, which have been engineered to carry genes identified as stated above. Examples of suitable plants include vegetable and oilseed plants, such as *B. napus, Vitis vinifera* (grape vine), *Zea mays* (corn=maize, *Glycine max* (soyabean), and *Helianthus* (sunflower). Especially preferred transgenic plants of the invention are oilseed *Brassica* species such as *B. napus.*

In addition to a plant, the present invention provides any clone of such a plant, seed, selfed or hybrid progeny and descendants, and any part of any of these, such as cuttings, seed. The invention provides any plant propagule that is any part which may be used in reproduction or propagation, sexual or asexual, including cuttings, seed and so on. Also encompassed by the invention is a plant which is a sexually or asexually propagated off-spring, clone or descendant of such a plant, or any part or propagule of said plant, off-spring, clone or descendant.

The present invention also encompasses the polypeptide expression product of a nucleic acid molecule according to the invention as disclosed herein or obtainable in accordance with the information and suggestions herein. Also provided are methods of making such an expression product by expression from a nucleotide sequence encoding therefore under suitable conditions in suitable host cells e.g. *E.coli*. Those skilled in the art are well able to construct vectors and design protocols and systems for expression and recovery of products of recombinant gene expression.

A polypeptide according to the present invention may be an allele, variant, fragment, derivative, mutant or homologue of the(a) polypeptides as mentioned herein. The allele, variant, fragment, derivative, mutant or homologue may have substantially the same function of the polypeptides alluded to above and as shown herein or may be a functional mutant thereof.

"Homology" in relation to an amino acid sequence or polypeptide sequence produced by the method of the invention may be used to refer to identity or similarity, preferably identity. As noted already above, high level of amino acid identity may be limited to functionally significant domains or regions.

In certain embodiments, an allele, variant, derivative, mutant derivative, mutant or homologue of the specific sequence may show little overall homology, say about 20%, or about 25%, or about 30%, or about 35%, or about 40% or about 45%, with the specific sequence. However, in functionally significant domains or regions, the amino acid homology may be much higher. Putative functionally significant domains or regions can be identified using processes of bioinformatics, including comparison of the sequences of homologues.

Functionally significant domains or regions of different polypeptides may be combined for expression from encoding nucleic acid as a fusion protein. For example, particularly advantageous or desirable properties of different homologues may be combined in a hybrid protein, such that the resultant expression product, may include fragments of various parent proteins, if appropriate.

Similarity of amino acid sequences may be as defined and determined by the TBLASTN program, of Altschul et al. (1990) J. Mol. Biol. 215: 403-10, which is in standard use in the art. In particular, TBLASTN 2.0 may be used with Matrix BLOSUM62 and GAP penalties: existence: 11, extension: 1. Another standard program that may be used is BestFit, which is part of the Wisconsin Package, Version 8, September 1994, (Genetics Computer Group, 575 Science Drive, Madison, Wisconsin, USA, Wisconsin 53711). BestFit makes an optimal alignment of the best segment of similarity between two sequences. Optimal alignments are found by inserting gaps to maximize the number of matches using the local homology algorithm of Smith and Waterman (Adv. Appl. Math. (1981)2: 482-489). Other algorithms include GAP, which uses the Needleman and Wunsch algorithm to align two complete sequences that maximizes the number of matches and minimizes the number of gaps. As with any algorithm, generally the default parameters are used, which for GAP are a gap creation penalty = 12 and gap extension penalty = 4. Alternatively, a gap creation penalty of 3 and gap extension penalty of 0.1 may be used. The algorithm FASTA (which uses the method of Pearson and Lipman (1988) PNAS USA 85: 2444-2448) is a further alternative.

Use of either of the terms "homology" and "homologous" herein does not imply any necessary evolutionary relationship between compared sequences, in keeping for example with standard use of terms such as "homologous recombination" which merely requires that two nucleotide sequences are sufficiently similar to recombine under the appropriate conditions. Further discussion of polypeptides according to the present invention, which may be encoded by nucleic acid according to the present invention, is found below.

The person skilled in the art will appreciate that further included within the ambit of the invention are methods of producing a host cell of the invention as provided herein, wherein the method includes incorporating polynucleotides of use in the invention or nucleic acid vectors of use in the invention into the cell by means of transformation. Such methods include regenerating a plant from a cell from one or more transformed cells.

Plants of the invention comprise a plant cell as herein described and include plants comprising plant cells as herein described and include plants selected from the group consisting of vegetable and oilseed *Brassica* species, zea mays (maize=corn), Glycine max (soyabean), and Helianthus species (sunflower).

Uses of polynucleotides in the invention are also included within the ambit of the invention. Such uses include use of a polynucleotide of use in the invention in the production of a transgenic plant; use of a polynucleotide as herein described in a selection process for a transformed germinating plant in the presence of a substituted pyridine ring comprising a halogen substituent; use of a polynucleotide as herein described wherein the substituted pyridine ring is selected from the group fluorouridine, fluorouracil, and fluoroorotic acid; use of a polynucleotide as described herein as a selectable marker in a plant that is germinating in the presence of a substituted pyridine ring comprising a halogen substituent wherein the said substituted pyridine ring is in contact with the plant.

Also included within the ambit of the invention is a method of selecting for a transformed plant that comprises growing a germinating plant carrying a polynucleotide as described herein wherein the polynucleotide is expressed in the germinating plant and the said plant is in the presence of a substituted pyridine ring comprising a halogen substituent, for example wherein the substituted pyridine ring is selected from the group fluorouridine, fluorouracil, and fluoroorotic acid.

The following preferred embodiments of the invention are included equally:
1. A method of producing a transient tolerance in a plant cell to an externally applied chemical stimulus that comprises:
   1) introducing into the said plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said plant cell is capable of providing a tolerance to the said externally applied chemical stimulus to the said plant cell;
   2) growing said plant cell of (1) under conditions wherein said non-constitutive plant nuclear promoter drives expression of said isolated nucleic acid sequence;
   3) selecting a plant cell of (2) wherein said isolated nucleic acid sequence is integrated into the nuclear genome;
   4) growing the plant cell of (3) under conditions wherein said non-constitutive plant promoter drives the expression of said heterologous or exogenous polynucleotide sequence from said nucleic acid sequence.
2. A method of producing at least a heterologous or exogenous polynucleotide sequence that is capable of providing a tolerance to a plant to an externally applied chemical stimulus that comprises:
   1) introducing into a regenerable plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said regenerable plant cell is capable of providing a tolerance to the said externally applied chemical stimulus to the said regenerable plant cell;
   2) growing said plant cell of (1) under conditions wherein said non-constitutive plant nuclear promoter drives expression of said isolated nucleic acid sequence;
   3) selecting a plant cell of (2) wherein said isolated nucleic acid sequence is integrated into the nuclear genome;
   4) regenerating a plant from the plant cell of (3); and
   5) growing the plant of (4) under conditions wherein said non-constitutive promoter expresses said heterologous or exogenous polypeptide from said isolated nucleic acid sequence.
3. A method according to embodiment 1 or embodiment 2 wherein the heterologous or exogenous polynucleotide sequence is selected from the group of polynucleotide sequences that code for functionally active proteins that confer tolerance to plant cells to spectinomycin, streptomycin, kanamycin, neomycin, hygromycin, puramycin, phosphinotricin, chlorsulfuron, methotrexate, gentamycin, imidazolinones, glyphosate, and pyridine ring structures substituted with halogen such as fluorouridine, fluoroorotic acid and fluorouracil.
4. A method according to embodiment 3 wherein the heterologous or exogenous polynucleotide sequence is a nucleosidase sequence that confers tolerance to plant cells in the presence of a substituted pyridine ring structure substituted with halogen such as fluorouridine, fluoroorotic acid and fluorouracil.
5. A method according to any one of embodiments 1 to 4 wherein the polynucleotide sequence is a uridine ribohydrolase 1 sequence or a functional part thereof.
6. A method according to any one of embodiments 1 to 5 wherein the non-constitutive promoter is selected from tissue specific promoters that are operative in plant cells of a plant in its germination phase.
7. A method according to embodiment 6 wherein the promoter region is a plant nuclear promoter region that is selected from a plant malate synthase promoter region or a functional part thereof and a plant isocitrate lyase promoter region or a functional part thereof.
8. A method according to embodiment 7 wherein the plant nuclear promoter region is the malate synthase promoter region of a plant or a functional part thereof.
9. A method according to embodiment 7 or embodiment 8 wherein the promoter region is selected from the *Arabidopsis thaliana* malate synthase promoter (At5g03860)(SEQ ID No.3) or a functional part thereof and the Arabidopsis thaliana isocitrate lyase promoter (At3g21720)(SEQ ID No.2) or a functional part thereof.
10. A method according to any one of embodiments 1 to 9 wherein the said isolated nucleic acid sequence codes for a functionally active protein that is a plant uridine ribohydrolase that codes for a functionally active uridine ribohydrolase protein.
11. A method according to any one of embodiments 1 to 10 wherein the said nucleic acid sequence further includes a second isolated nucleic acid sequence that when expressed provides a protein selected from an herbicide tolerance protein or a functionally active part thereof or an antibiotic tolerance protein, or a functionally active part thereof.
12. A method according to embodiment 10 or embodiment 11 wherein the plant uridine ribohydrolase nucleic acid sequence is one corresponding to a functionally active protein selected from the *Arabidopsis thaliana* sequence At2g36310, the *Zea mays* sequence ACF80359, the *Oryza sativa* sequence Os08g05579;XP483754, and the *Vitis vinifera* sequence, CAO15947.
13. A method according to any one of the preceding embodiments wherein the heterologous or exogenous polypeptide is at least one selected from the group consisting of the *Arabidopsis thaliana* sequence At2g36310 or a functionally active part thereof, the Zea *mays* sequence ACF80359 or a functionally active part thereof, the *Oryza sativa* sequence Os08g05579;XP483754 or a functionally active part thereof, and the *Vitis vinifera* sequence, CAO15947, or a functionally active part thereof.
14. A method according to any one of the preceding embodiments wherein the externally applied chemical stimulus is a substituted pyridine ring comprising a halogen.
15. A method according to embodiment 14 wherein the substituted pyridine ring is selected from the group fluorouridine, fluorouracil, and fluoroorotic acid.
16. An isolated polynucleotide that encodes an heterologous or exogenous polynucleotide sequence operably linked to a non-constitutive plant nuclear promoter that drives expression in a plant cell that is selected from the group of polynucleotide sequences that code for functionally active proteins that confer tolerance to plant cells to spectinomycin, streptomycin, kanamycin, neomycin, hygromycin, puramycin, phosphinotricin, chlorsulfuron, methotrexate, gentamycin, imidazolinones, glyphosate, and pyridine ring structures substituted with halogen such as fluorouridine, fluoroorotic acid and fluorouracil which is suitable for use in a method according to any one of embodiments 1 to 15.
17. An isolated polynucleotide that encodes an heterologous or exogenous polynucleotide sequence that is a nucleosidase nucleic acid sequence that comprises a first isolated nucleosidase nucleic acid sequence operably linked to a non-constitutive plant nuclear promoter that drives expression in a plant cell wherein the said nucleosidase nucleic acid sequence is selected from nucleosidase nucleic acid sequences that are substantially operative during the germination stages of a plant and which is suitable for use in a method according to any one of embodiments 1 to 16.
18. An isolated polynucleotide according to embodiment 17 wherein the nucleosidase nucleic acid sequence is operably linked to a malate synthase promoter or a functional part thereof, or is operably linked to an isocitrate lyase promoter or a functional part thereof.
19. An isolated polynucleotide according to embodiment 17 or embodiment 18 wherein the said nucleosidase nucleic acid sequence is a uridine ribohydrolase nucleic acid sequence from a plant for use in a method according to any one of embodiments 1 to 15.
20. An isolated nucleic acid sequence according to embodiment 19 wherein the nucleosidase nucleic acid sequence is a plant uridine ribohydrolase nucleic acid sequence that is one that encodes a functionally active protein selected from the *Arabidopsis thaliana* sequence At2g36310 or functionally active parts thereof, the Zea *mays* sequence ACF80359 or functionally active parts thereof, the *Oryza sativa* sequence Os08g05579;XP483754 of functionally active parts thereof, and the *Vitis vinifera* sequence CAO15947 or functionally active parts thereof.
21. An isolated polynucleotide sequence according to any one of embodiments 16 to 20 comprising genomic DNA.
22. An isolated polynucleotide sequence according to any one of embodiments 16 to 20 comprising a cDNA component.
23. A nucleic acid vector suitable for transformation of a plant cell or a bacterial cell and including a polynucleotide according to any one of embodiments 16 to 22.
24. A nucleic acid vector according to embodiment 23 suitable for transformation of a prokaryotic cell and including a polynucleotide according to any one of embodiments 16 to 22.
25. A nucleic acid vector according to embodiment 24 suitable for transforming an *Agrobacterium* cell.
26. A nucleic acid vector according to embodiment 25 suitable for transforming a plant cell of an oil seed bearing plant.
27. A nucleic acid vector according to embodiment 26 that is suitable for transforming a plant cell of an oil seed bearing plant selected from the group oilseed bearing *Brassicaceae* species, such as *Brassica napus, Zea mays* (maize=corn), *Glycine max* (soyabean), and *Helianthus* species (sunflower).
28. A host cell containing a heterologous polynucleotide or nucleic acid vector according to any one of embodiments 16 to 27.
29. A host cell according to embodiment 28 which is a plant or a bacterial cell.
30. A host cell according to embodiment 28 or embodiment 29 wherein the plant cell is selected from a plant from the group oilseed bearing *Brassicaceae* species, such as *Brassica napus, Zea mays* (maize=corn), *Glycine max* (soyabean), and *Helianthus* species (sunflower).
31. A host cell according to any one of embodiments 28 to 30 that is comprised in a plant, a plant part or a plant propagule, or in a plant cell culture.
32. A method of producing a cell according to any of embodiments 28 to 31, the method including incorporating said polynucleotide or nucleic acid vector into the cell by means of transformation.
33. A method according to embodiment 32 which includes regenerating a plant from a cell according to any of embodiments 28 to 31 from one or more transformed cells.
34. A plant comprising a plant cell according to any one of embodiments 28 to 31.
35. A plant comprising a plant cell according to embodiment 34 that is selected from the group consisting of vegetable and oilseed *Brassicaceae* species, zea mays (maize=corn), Glycine max (soyabean), and Helianthus species (sunflower).
36. A method of producing a plant, the method including incorporating a polynucleotide or nucleic acid vector according to any of embodiments 16 to 27 into a plant cell and regenerating a plant from said cell.
37. Use of a polynucleotide according to any one of embodiments 16 to 22 in the production of a transgenic plant.
38. Use of a polynucleotide according to any one of embodiments 16 to 22 in a selection process for a transformed germinating plant in the presence of a substituted pyridine ring comprising a halogen substituent.
39. Use according to embodiment 38 wherein the substituted pyridine ring is selected from the group fluorouridine, fluorouracil, and fluoroorotic acid.
40. Use of a polynucleotide according to any of embodiments 16 to 22 as a selectable marker in a plant that is germinating in the presence of a substituted pyridine ring comprising a halogen substituent wherein the said substituted pyridine ring is in contact with the plant.
41. Use according to embodiment 40 wherein the substituted pyridine ring is selected from the group fluorouridine, fluorouracil, and fluoroorotic acid.
42. A method of selecting for a transformed plant that comprises growing a germinating plant carrying a polynucleotide according to any of embodiments 16 - 22 wherein the polynucleotide is expressed in the germinating plant and the said plant is in the presence of a substituted pyridine ring comprising a halogen substituent.
43. A method according to embodiment 42 wherein the substituted pyridine ring is selected from the group fluorouridine, fluorouracil, and fluoroorotic acid.

The teaching of all references cited herein is incorporated in its entirety into the present description.

There now follow non-limiting examples and figures illustrating the invention.
**Figure 1****:** Uridine ribohydrolase cDNA nucleic acid sequence.

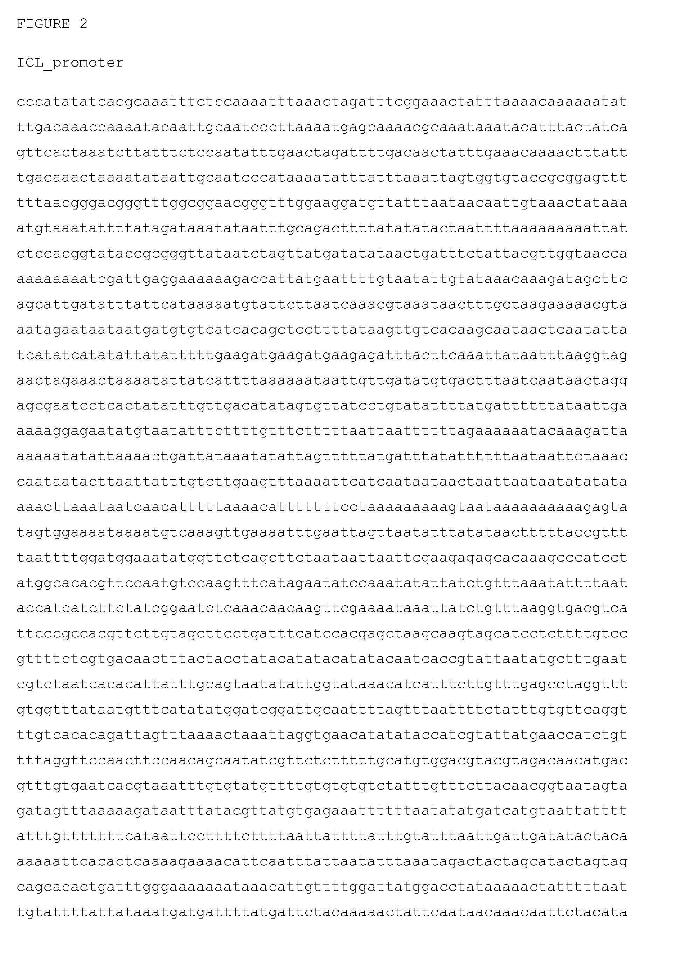

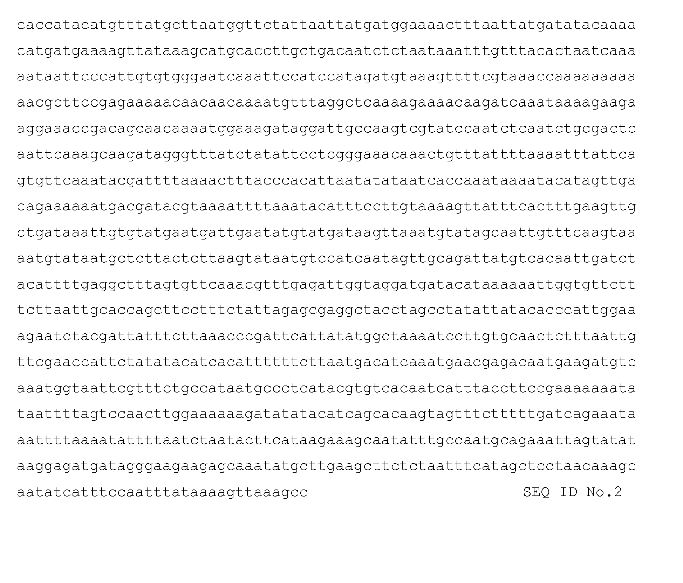

**Figure 2****:** Isocitrate Lyase promoter cDNA nucleic acid sequence.
**Figure 3****:** Malate Synthase promoter cDNA nucleic acid sequence.
**Figure 4****:** Map of pBatL_promMLS.

**Spec:** spectinomycin resistance
**LB:** left border
**BaR:** BASTA® resistance
**promoter MLS:** malate synthase promoter
**RB**: right border
**Chloramphenicol:** chloramphenicol resistance
**ccdB:** coding for ccdB protein inhibiting growth of non-resistant bacteria strains
**attR1/attR2:** Gateway® recombination sites

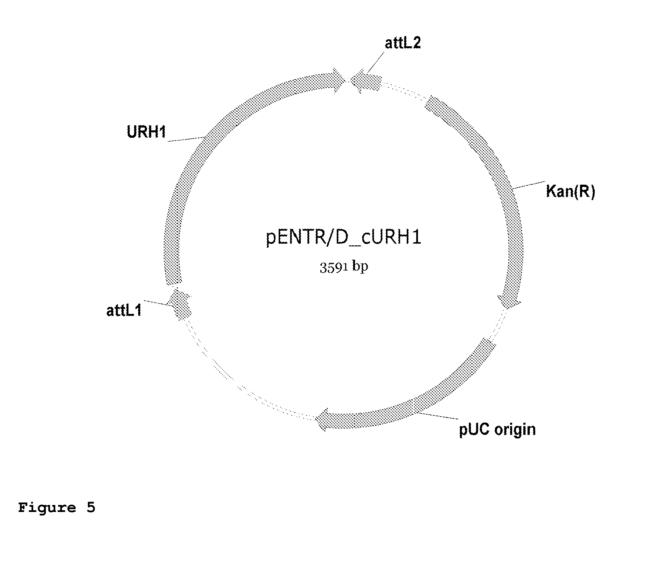

**Figure 5****:** Map of pENTR/D_cURH1.
**Kan(R):** kanamycin resistance
**URH1:** coding region of URH1
**pUC origin:** replication origin
**attL1/attL2:** Gateway® recombination sites

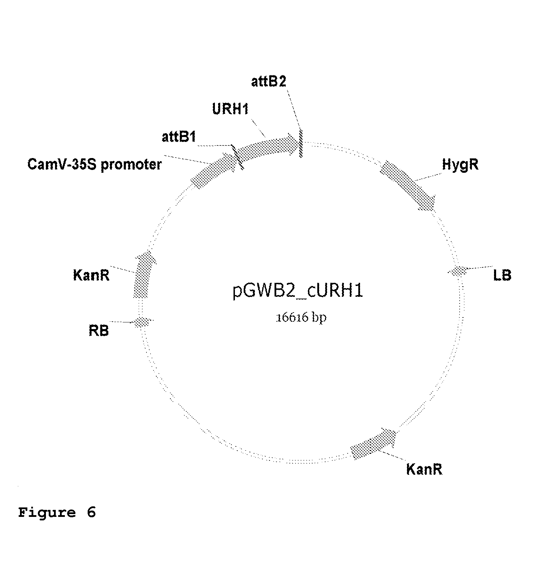

**Figure 6****:** Map of pGWB2_cURH1.
**HygR:** hygromycin resistance
**LB:** left border
**KanR:** kanamycin resistance
**RB:** right border
**URH1**: coding region of URH1
**CamV-35S promoter**: cauliflower mosaic virus promoter
**attB1/attB2:** Gateway® recombination sites

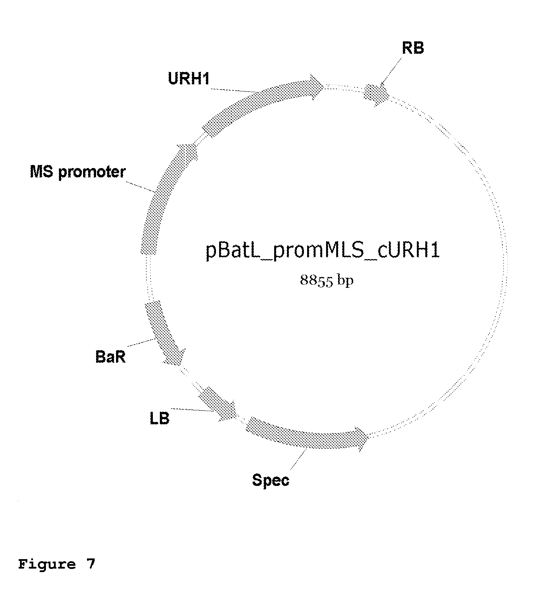

**Figure 7****:** Map of pBatL_promMLS_cURH1.
**URH1:** URH1 coding region
**LB:** left border
**Ms Promoter**: malate synthase promoter
**Spec:** spectinomycin resistance
**BaR:** BASTA®resistance
**RB:** right border

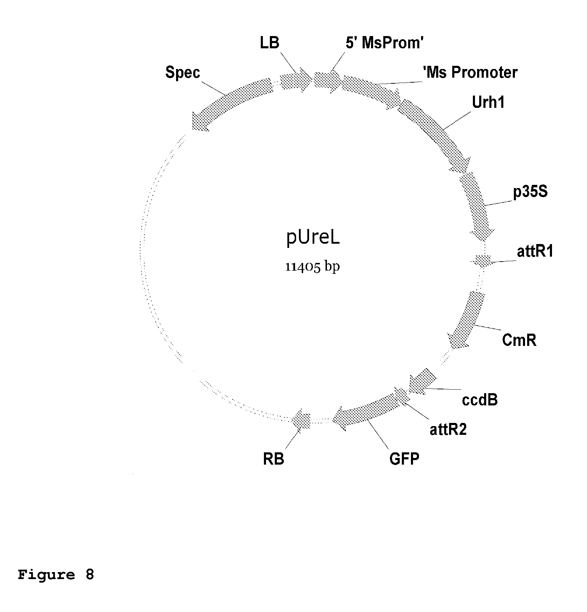

**Figure 8****:** Map of pUreL.
**Spec:** spectinomycin resistance
**LB:** left border
**5' MSprom'**: short PCR amplified end of malate synthase promoter **'Ms Promoter**: short version of malate synthase promoter **5'** MSprom' and Ms Promoter together represent full length malate synthase promoter used in pGWB2_cURH1 with mutated AseI restriction site
**URH1:** URH1 coding region
**p35S:** cauliflower mosaic virus promoter
**CmR:** chloramphenicol resistance
**RB:** right border
**ccdB**: coding for ccdB protein inhibiting growth of non-resistant bacteria strains
**GFP:** green fluorescent protein coding region
**attR1/attR2:** Gateway® recombination sites

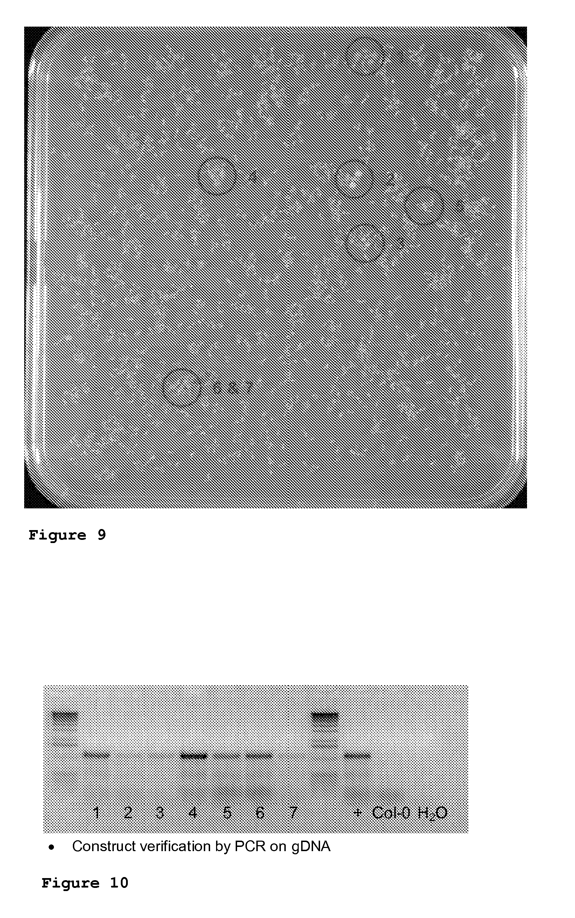

**Figure 9****:** Identification of 5-FD resistant seedlings harbouring T-DNA from pGWB2_cURH1.
10 days of growth on agar plates without sucrose and 50µM 5-FD in long day conditions (16/8h day/night) at 22°C
Circles mark resistant seedlings, numbers correspond in Figure 10

**Figure 10****:** Verification of construct presence in plants displayed in Figure 9.
Presence of a band indicates integration of construct into plant genomic DNA (1-7)
**+**: positive control (plasmid DNA)

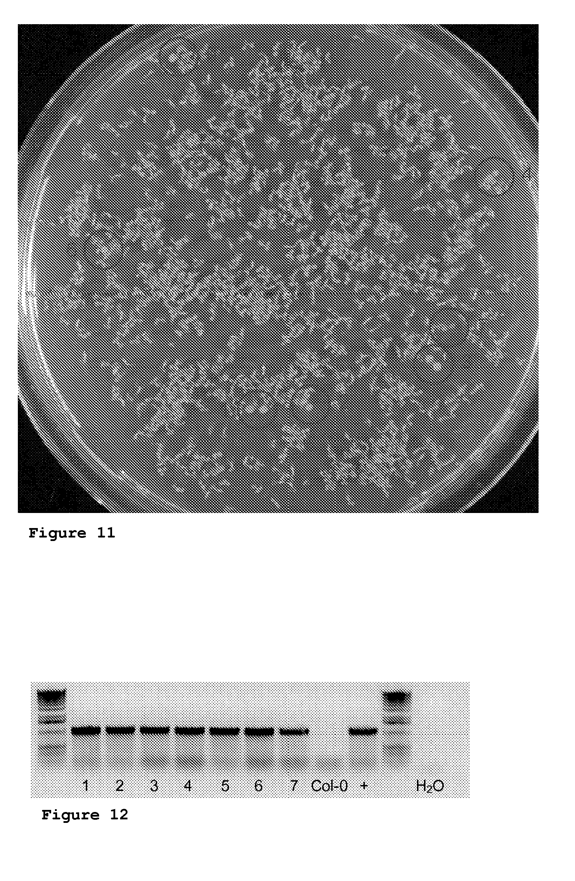

**Figure 11****:** Identification of 5-FD resistant seedlings harbouring T-DNA from pBatL_promMLS_cURH1.
5 days of growth on agar plates without sucrose and 50µM 5-FD in long day conditions (16/8h day/night) at 22°C
Circles mark resistant seedlings, numbers correspond in Figure 12

**Figure 12****:** Verification of construct presence in plants displayed in Figure 11.
Presence of a band indicates integration of construct into plant genomic DNA (1-7)
**+**: positive control (plasmid DNA)
Col-0 (wild-type gDNA) and H₂O: negative control

### Examples Section

### Isolation of malate synthase promoter (promMLS)

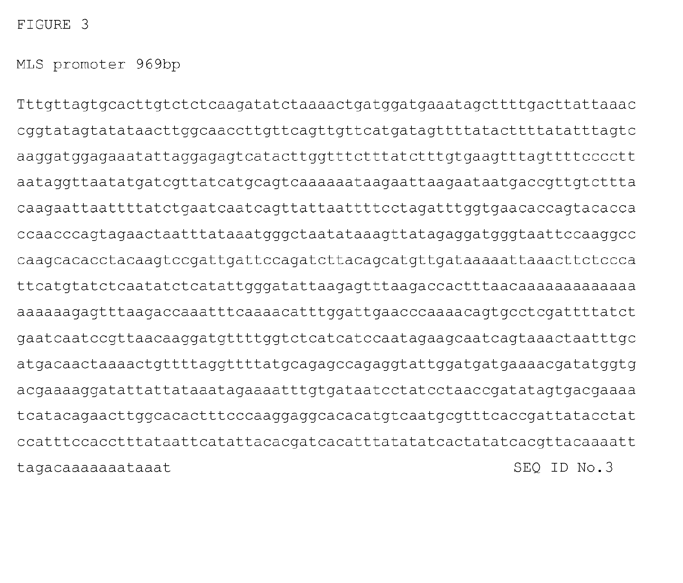

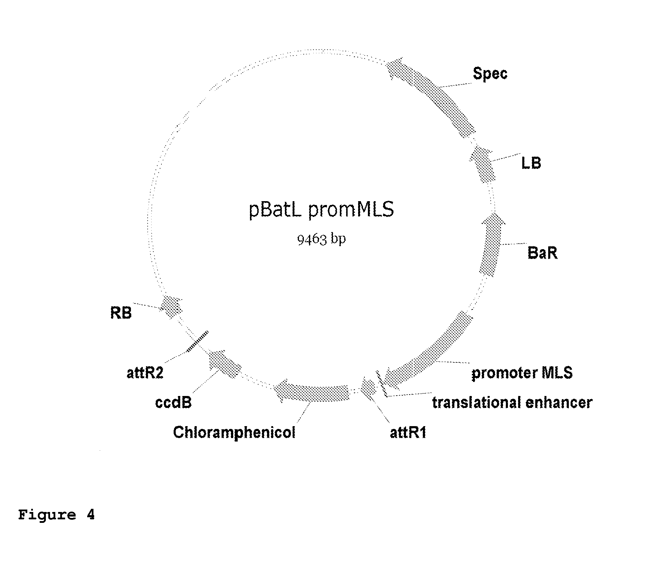

**T**he malate synthase promoter (Figure 3) was amplified by PCR from *Arabidopsis thaliana (A. th*.) genomic DNA using 5'-phosphorylated primers *MS prom fwd* (5'-TTTGTTAGTGCACTTGTCTCTC-3') (SEQ ID No.4) and *MS prom rev* (5'-ATTTATTTTTTTGTCTAAATTTTGTAACG-3') (SEQ ID No. 5) applying a proofreading DNA polymerase (Pfu Ultra, Stratagene, La Jolla, USA). The resulting PCR product of 969 bp was ligated (T4-ligase, Fermentas, St. Leon-Rot, Germany) blunt-end into the EcoRV-opened vector pBatL (Figure 4) to give rise to pBatL_promMLS. Sequence integrity of promMLS in the resulting vector was verified through dye-terminator sequencing and capillary electrophoresis (BigDye®, Applied Biosystems).

### Isolation of uridine hydrolase 1 (URH1) cDNA

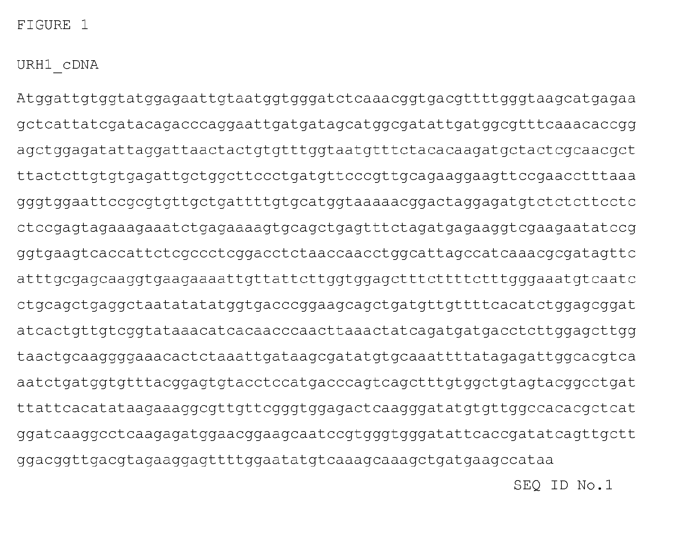

A complementary DNA (cDNA) prepared by reverse transcription (Bioscript...) of messenger RNA (mRNA) extracted from mature *A.th*. leaf tissue was used as template to amplify the coding region of uridine hydrolase 1 (URH1, At2g36310, Figure 1) in a PCR reaction. The PCR reaction was performed using primers *URH1 TOPO fwd* (5'-CACCATGGATTGTGGTATGGAG-3') (SEQ ID No. 6) and *URH1 rev* (5'-TTATGGCTTCATCAGCTTTGC-3') (SEQ ID No. 7) using a proofreading DNA polymerase (Pfu Ultra, Stratagene, La Jolla, USA). The resulting PCR product of 1015 bp was ligated into the pENTR/D^{®} vector (Invitrogen, Carlsbad, USA) using the TOPO^{®} cloning kit (Invitrogen, Carlsbad, USA), giving rise to pENTR/D_cURH1 (Figure 5). DNA sequence integrity was subsequently verified to match the published sequence (At2g36310, Figure 1) through dye-terminator sequencing (BigDye^{®}, Applied Biosystems, Foster City, USA) and capillary electrophoresis.

### Generation of cauliflower mosaic virus promoter (CaMV 35S) driven URH1

The URH1 coding region from pENTR/D_cURH1 was recombined into the pGWB2 destination vector via LR-reaction using the LR-clonase kit (Invitrogen, Carlsbad, USA). The resulting plasmid (pGWB2_cURH1, Figure 6) was transformed into *Agrobacterium tumefaciens* and transformants were selected based on kanamycin and hygromycin (conferred by pGWB2) and rifampicin and gentamycin (intrinsic to *Agrobacterium tumafaciens* strain GV3101) resistance. The transfer DNA (T-DNA) of pGWB2_cURH1 was then transformed into ecotype Columbia-0 *Arabidopsis thaliana* by the floral dip method and Agrobacterium-mediated transformation.

Transformed plants were selected based on 5-fluor uridine resistance (50µM) conferred by constitutive over expression of uridine ribohydrolase (Figure 9). Plants selected as transformants were transferred to soil and subjected to genotyping by genomic DNA PCR using primers *MS prom fwd* and *MS prom rev* (Figure 10).

### Generation of malate synthase promoter-driven URH1 (pBatL_promMLS_cURH1)

The URH1 coding region from pENTR/D_cURH1 was recombined into pBatL_promMLS using the LR-cloning kit (Invitrogen, Carlsbad, USA) to give rise to pBatL_promMLS_cURH1 (Figure 7). *Agrobacterium tumefaciens* strain GV3101 was transformed with pBatL_promMLS_cURH1 and transformants were selected based on spectinomycin (conferred by pBatL) and rifampicin and gentamycin (intrinsic to *Agrobacterium tumefaciens* strain GV3101) resistance. The transfer DNA (T-DNA) of pBatL_promMLS_cURH1 was then introduced into ecotype Columbia-0 *Arabidopsis thaliana* by Agrobacterium-mediated transformation applying the floral dip method. Transformed plants were selected based on 5-fluor uridine resistance (50µM) conferred through transient over expression of uridine ribohydrolase driven by promMLS activity (Figure 11). Plants selected as transformants were transferred to soil and subjected to genotyping by genomic DNA PCR using primers *MS prom fwd* and *MS prom rev* (Figure 12). All selected plants harbored a transfer DNA (T-DNA) containing cURH1 providing evidence for the suitability of transient URH1 over-expression as a selection marker for transformed plants.

### Generation of pUreL plasmid

In order to provide a ready-to-use, Gateway^{®} compatible vector for transferring the construct of interest into plants and using 5-FD resistance as selection marker, we generated the plasmid pUreL. Users may recombine their construct of interest from an appropriate Entry^{®} clone into the attR-sites of pUreL and select transformed plants based of 5-FD resistance during germination.

All PCR reactions were performed using a proofreading DNA polymerase (Pfu Ultra, Stratagene,). The coding region of URH1 was amplified by PCR from pENTR/D_cURH1 using primers *Ms Prom Urh1 fusion fwd* (5'-CGTTACAAAATTTAGACAAAAAAATAAATATGGATTGTGGTATGGAG-3') (SEQ ID No.8) and *Urh1 Stop BspHI rev* (5'-CTCATGAATTTATGGCTTCATCAGCTTTGC-3') (SEQ ID No.9). The malate synthase promoter was amplified by PCR from pBatL_promMLS using primers *MS prom fwd* (5'-TTTGTTAGTGCACTTGTCTCTC-3') (SEQ ID No.4) and *Ms Prom Urhl fusion rev* (5'-CTCCATACCACAA-TCCATATTTATTTTTTTGTCTAAATTTTGTAACG-3') SEQ ID No.10). Each PCR product was gel purified and both were pooled and used as template in a subsequent PCR reaction fusing both products due to 5' and 3' overhangs compatible with 3' end of promMLS and 5'-end of cURH1 respectively. Fusion PCR primers used were *MS prom fwd* (5'-TTTGTTAGTGCACTTGTCTCTC-3') (SEQ ID No. 4) and *Urh1 Stop BspHI rev* (5'-CTCATGAATTTATGGCTTCATCAGCTTTGC-3') (SEQ ID. No.9). The fusion PCR product comprising promMLS and cURH1 was cloned into pJet 1.2 (Fermentas, St. Leon-Rot, Germany) resulting in pJet_promMLS_cURH1 and sequence integrity was verified by dye-terminator sequencing and capillary electrophoresis. The intrinsic kanamycin resistance cassette of pBatL was replaced with promMLS_cURH1 by digestion with AseI / BspHI restriction endonuclease digestion and ligation with the AseI / BspHI restriction endonuclease digestion-fragment released from pJet_promMLS_cURH1. Since the malate synthase promoter contained two AseI restriction endonuclease sites 26 bp apart a promMLS fragment shortened by 305 bp at the 5'-end was initially ligated into pBatL. The short promMLS fragment was amplified by PCR using primers *MsProm_AseI_fwd* (5'-ATTAATTTTGTTAGTGCACTTGTCTCTC-3') (SEQ ID No.11) and *MsProm_-AseI_rev* (5'-ATTAATAACTGATTGATTCAGATAAAC-3')(SEQ ID No.12) from pJet_promMLS-cURH1. The forward primer *(MsProm_AseI_fwd)* contained an *AseI* site 5'-addition and the intrinsic second AseI site in the reverse primer (*MsProm_-AseI*_*rev)* was removed by changing A to C in the primer sequence. The resulting PCR product was digested with AseI and ligated into the AseI opened pBatL harboring the shortened promMLS_cURH1 giving rise to pUreL (Figure 8), comprising the full length malate synthase promoter and the coding region of URH1.

## Claims

1. A method of producing a transient tolerance in a plant cell to an externally applied chemical stimulus that comprises:
1) introducing into the said plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said plant cell is capable of providing a tolerance to the said externally applied chemical stimulus to the said plant cell;
2) growing said plant cell of (1) under conditions wherein said non-constitutive plant nuclear promoter drives expression of said isolated nucleic acid sequence;
3) selecting a plant cell of (2) wherein said isolated nucleic acid sequence is integrated into the nuclear genome;
4) growing the plant cell of (3) under conditions wherein said non-constitutive plant promoter drives the expression of said heterologous or exogenous polynucleotide sequence from said nucleic acid sequence.

2. A method of producing at least a heterologous or exogenous polynucleotide sequence that is capable of providing a tolerance to a plant to an externally applied chemical stimulus that comprises:
1) introducing into a regenerable plant cell a first nucleic acid sequence that comprises a non-constitutive plant nuclear promoter that is operable during the germination stages of a plant wherein the said non-constitutive plant nuclear promoter is operably linked to an isolated nucleic acid sequence that encodes an heterologous or exogenous polynucleotide sequence that when expressed as a polypeptide in the said regenerable plant cell is capable of providing a tolerance to the said externally applied chemical stimulus to the said regenerable plant cell;
2) growing said plant cell of (1) under conditions wherein said non-constitutive plant nuclear promoter drives expression of said isolated nucleic acid sequence;
3) selecting a plant cell of (2) wherein said isolated nucleic acid sequence is integrated into the nuclear genome;
4) regenerating a plant from the plant cell of (3); and
5) growing the plant of (4) under conditions wherein said non-constitutive promoter expresses said heterologous or exogenous polypeptide from said isolated nucleic acid sequence.

3. A method according to claim 1 or claim 2 wherein the heterologous or exogenous polynucleotide sequence is selected from the group of polynucleotide sequences that code for functionally active proteins that confer tolerance to plant cells to spectinomycin, streptomycin, kanamycin, neomycin, hygromycin, puramycin, phosphinotricin, chlorsulfuron, methotrexate, gentamycin, imidazolinones, glyphosate, and pyridine ring structures substituted with halogen such as fluorouridine, fluoroorotic acid and fluorouracil.

4. A method according to claim 3 wherein the heterologous or exogenous polynucleotide sequence is a nucleosidase sequence that confers tolerance to plant cells in the presence of a substituted pyridine ring structure substituted with halogen such as fluorouridine, fluoroorotic acid and fluorouracil.

5. A method according to any one of claims 1 to 4 wherein the polynucleotide sequence is a uridine ribohydrolase 1 sequence or a functional part thereof.

6. A method according to any one of claims 1 to 5 wherein the non-constitutive promoter is selected from tissue specific promoters that are operative in plant cells of a plant in its germination phase.

7. A method according to claim 6 wherein the promoter region is a plant nuclear promoter region that is selected from a plant malate synthase promoter region or a functional part thereof and a plant isocitrate lyase promoter region or a functional part thereof.

8. A method according to claim 7 wherein the plant nuclear promoter region is the malate synthase promoter region of a plant or a functional part thereof.

9. An isolated polynucleotide that encodes an heterologous or exogenous polynucleotide sequence that is a nucleosidase nucleic acid sequence that comprises a first isolated nucleosidase nucleic acid sequence operably linked to a non-constitutive plant nuclear promoter that drives expression in a plant cell wherein the said nucleosidase nucleic acid sequence is selected from nucleosidase nucleic acid sequences that are substantially operative during the germination stages of a plant and which is suitable for use in a method according to any one of claims 1 to 8.

10. An isolated polynucleotide according to claim 9 wherein the nucleosidase nucleic acid sequence is operably linked to a malate synthase promoter or a functional part thereof, or is operably linked to an isocitrate lyase promoter or a functional part thereof.

11. A nucleic acid vector suitable for transformation of a plant cell or a bacterial cell and including a polynucleotide according to claim 9 or claim 10.

12. A host cell containing a heterologous polynucleotide or nucleic acid vector according to any one of claims 9 to 11.

13. A host cell according to claim 12 wherein the plant cell is selected from a plant from the group oilseed bearing *Brassicaceae* species, such as *Brassica napus, Zea mays* (maize=corn), *Glycine max* (soyabean), and *Helianthus* species (sunflower).

14. Use of a polynucleotide according to claim 9 or claim 10 as a selectable marker in a plant that is germinating in the presence of a substituted pyridine ring comprising a halogen substituent wherein the said substituted pyridine ring is in contact with the plant.

15. A method of selecting for a transformed plant that comprises growing a germinating plant carrying a polynucleotide according to claim 9 or claim 10 wherein the polynucleotide is expressed in the germinating plant and the said plant is in the presence of a substituted pyridine ring comprising a halogen substituent.
